# EUROPÄISCHE PATENTANMELDUNG

(11) **EP 4 796 065 A1**
(43) Veröffentlichungstag der Anmeldung: **26.08.2026**
(21) Anmeldenummer: 25159859.5
(22) Anmeldetag: 25.02.2025
(51) Int. Cl.: A61B 34/30, A61B 18/12, A61B 90/00, A61B 18/14

(54) **CHIRURGIESYSTEM**

(71) Anmelder: Erbe Elektromedizin GmbH, 72072 Tübingen (DE)
(72) Erfinder: Selig, Peter, 72147 Nehren (DE); Linzenbold, Walter, 71034 Böblingen (DE); Ederer, Michael, 70563 Stuttgart (DE)
(74) Vertreter: Rüger Abel IP Legal Solutions GmbH

(57) **Zusammenfassung**

Die Erfindung betrifft ein Chirurgiesystem (10), aufweisend eine Vorrichtung (11) und einen Roboter (12), sowie ein Verfahren zu dessen Betrieb. Die Vorrichtung (11) weist ein Versorgungsgerät (20) und wenigstens ein an das Versorgungsgerät (20) angeschlossenes Instrument (21) zur Behandlung von biologischem Gewebe (G)auf. Das Instrument (21) ist an eine Halteeinrichtung (44) des Roboters (12) angeordnet und kann mittels des Roboters (12) relativ zu dem zu behandelnden Gewebe (G) positioniert und/oder bewegt werden, wobei diese Handhabung durch wenigstens einen Handhabungsparameter (PH) beschrieben wird. Zumindest einer der Handhabungsparameter (PH) oder mehrere Handhabungsparameter (PH) werden der Vorrichtung (11) bereitgestellt, die abhängig von einem oder mehreren Handhabungsparametern (PH) einen oder mehrere Betriebsparameter (PB) für den Betrieb des Instruments (21) ermittelt und steuert oder regelt. Dadurch ist eine optimale Anpassung des Betriebs des Instruments (21) an die aktuell mittels des Roboters (12) auf das Instrument (21) ausgeübte Bewegung bzw. Kraft erreicht.

## Beschreibung

Die Erfindung betrifft ein Chirurgiesystem mit einem Roboter und einer Vorrichtung. Die Vorrichtung hat ein Versorgungsgerät sowie wenigstens ein an das Versorgungsgerät angeschlossenes Instrument, das beispielsweise ein elektrochirurgisches Instrument oder ein irgendein Instrument sein kann, das vom Versorgungsgerät mit wenigstens einem Betriebsmedium (z.B. Gas und/oder Flüssigkeit und/oder Ultraschall und/oder Licht und/oder elektrische Leistung) versorgt wird. Das Instrument dient zur Behandlung von biologischem Gewebe eines Patienten.

Der Roboter hat einen Roboterarm mit einer am Roboterarm angeordneten Halteeinrichtung. Die Halteeinrichtung ist zum lösbaren Anordnen des Instruments oder optional auch mehrerer Instrumente eingerichtet. Mittels des Roboters kann das wenigstens eine Instrument relativ zu dem biologischen Gewebe positioniert und/oder bewegt und/oder eine Kraft zwischen dem Instrument und dem biologischen Gewebe erzeugt werden.

Derartige Chirurgiesysteme mit einem Roboter, der das Instrument während der Behandlung handhabt beziehungsweise führt, können die Behandlung von biologischem Gewebe für eine Bedienperson (zum Beispiel Chirurg) vereinfachen. Beispielsweise offenbart WO 2014/004116 A1 ein Chirurgiesystem mit einem Roboter und einem elektrochirurgischen Instrument, das vom Roboter gehalten und bewegt wird. Eine Robotersteuerung kann dabei die Geschwindigkeit des Instruments an einen zu behandelnden Gewebetyp anpassen, wobei der Gewebetyp insbesondere anhand einer ermittelten Impedanz bestimmt werden kann.

Aus EP 2 854 665 A2 ist es bekannt, sensorisch erfasste Werte, beispielsweise eine Kraft oder eine Geschwindigkeit eines Instruments relativ zu einem behandelnden Gewebe zu messen und anhand solcher Handhabungsparameter die gewünschte Gewebebehandlungsart zu erkennen und einzustellen. Hierzu sind entsprechende Sensoren am elektrochirurgischen Instrument vorhanden.

In EP 3 795 106 B1 ist eine Token-basierte Steuerung beschrieben. Über einen Token werden eine Robotersteuerung eines Roboters und eine Steuerung eines HF-Generators, der ein elektrochirurgisches Instrument versorgt, miteinander koordiniert.

EP 4 132 407 A2 betrifft ein Chirurgiesystem, bei dem eine Klemmkraft eines zangenartigen Instruments, das von einem Roboterarm geführt wird, abhängig von der Impedanz des geklemmten Gewebes eingestellt werden kann.

Im dem Stand der Technik werden daher unterschiedliche Ansätze zur Koordination der Steuerung eines Roboters und dem Betrieb eines am Roboter gehaltenen Instruments beschrieben. Bei einer solchen Koordination ist es wesentlich, dass die Handhabung des Instruments mittels des Roboters und der Betrieb des Instruments zur Behandlung von biologischem Gewebe sehr genau aufeinander abgestimmt werden, um den gewünschten Behandlungseffekt zu erreichen. Dazu wurde beispielsweise vorgeschlagen, am Instrument zusätzliche Sensoren anzuordnen, um Informationen über die tatsächliche Position oder Bewegung des Instruments oder die vom Instrument auf das Gewebe ausgeübte Kraft zu erhalten. Zusätzliche Sensoren erhöhen nicht nur die Kosten und den Bauteilaufwand eines solchen Chirurgiesystems, sondern benötigen außerdem Bauraum, der wiederum die Einsatzmöglichkeiten des Instruments einschränken kann. Zusätzliche Sensoren können die Handhabung des Instruments bei der Behandlung von biologischem Gewebe erschweren oder behindern.

Es kann daher als Aufgabe der vorliegenden Erfindung angesehen werden, ein Chirurgiesystem zu schaffen, das bei einfachem konstruktivem Aufbau eine sehr gute zeitliche Koordination zwischen der Positionierung und/oder Bewegung des Instruments einerseits und der Versorgung des Instruments andererseits gewährleistet und insbesondere eine Koordination in Echtzeit oder mit geringer Latenz sicherstellt.

Diese Aufgabe wird durch ein Chirurgiesystem mit den Merkmalen des Patentanspruches 1 gelöst. Außerdem betrifft die Erfindung ein Verfahren mit den Merkmalen des Patentanspruches 14.

Das erfindungsgemäße Chirurgiesystem weist eine Vorrichtung auf. Die Vorrichtung hat ein Versorgungsgerät mit einer Gerätesteuerung. An das Versorgungsgerät ist wenigstens ein elektrochirurgisches Instrument angeschlossen oder anschließbar. Das Versorgungsgerät kann das angeschlossene Instrument oder zumindest eines der angeschlossenen Instrumente mit dem wenigstens einen für den Betrieb erforderlichen Betriebsmedium versorgen, beispielsweise mit elektrischer Leitung bzw. Energie versorgen. Zusätzlich oder alternativ kann das Versorgungsgerät auch ein Fluid (Flüssigkeit, Gas) oder eine Ultraschalleistung oder Licht als ein für den Betrieb von einem oder mehreren angeschlossenen Instrumenten erforderliches Betriebsmedium liefern (zum Beispiel Gas zur Plasmaerzeugung oder Wasser für ein Kryoinstrument, etc.). Dies Betriebsmedien können in beliebiger Kombination bereitgestellt werden.

Die Gerätesteuerung des Versorgungsgeräts ist dazu eingerichtet, wenigstens einen Betriebsparameter für das angeschlossene Instrument oder für zumindest eines der angeschlossenen Instrumente zu ermitteln und einzustellen (steuern oder regeln). Abhängig vom verwendeten Instrument kann es sich bei dem wenigstens einen Betriebsparameter beispielsweise um einen elektrischen Betriebsparameter und/oder fluidischen Betriebsparameter und/oder Ultraschall-Betriebsparameter und/oder Licht-Betriebsparameter, usw. handeln. Die Verbindung zwischen dem Versorgungsgerät und dem wenigstens einen angeschlossenen Instrument kann - abhängig von der Instrumentenkonfiguration - eine elektrische und/oder fluidische und/oder optische und/oder Ultraschall leitende Verbindung sein. Mittels einer optischen Verbindung kann beispielsweise Licht (zur Abstrahlung am Instrument und/oder am Instrument sensorisch erfasstes Licht) übertragen werden.

Jedes vorhandene elektrochirurgische Instrument kann beispielsweise eine Elektrode, zwei Elektroden oder auch mehr als zwei Elektroden aufweisen und dementsprechend als monopolares, bipolares oder multipolares Instrument ausgebildet sein. An die wenigstens eine Elektrode kann entsprechend einem elektrischen Betriebsparameter eine elektrische Spannung angelegt und/oder es kann ein elektrischer Strom in bzw. über die Elektrode fließen, wenn ein Behandlungsstromkreis geschlossen ist.

Wenn das elektrochirurgische Instrument ein monopolares Instrument ist oder als monopolares Instrument betrieben wird, kann eine an einem Patienten anzubringende Neutralelektrode elektrisch mit dem Versorgungsgerät verbunden sein. In diesem Fall kann der Behandlungsstromkreis vom Versorgungsgerät über das elektrochirurgische Instrument bzw. dessen Elektrode, das zu behandelnde biologische Gewebe des Patienten, die Neutralelektrode und von dort zurück zum Versorgungsgerät geschlossen werden. Wenn es sich bei dem elektrochirurgischen Instrument um ein bipolares oder multipolares Instrument handelt, kann der Behandlungsstromkreis vom Versorgungsgerät über eine Elektrode des elektrochirurgischen Instruments, das zu behandelnde biologische Gewebe, einer weiteren Elektrode des elektrochirurgischen Instruments und zurück zum Versorgungsgerät geschlossen werden.

Das Chirurgiesystem hat außerdem einen Roboter, der zur Handhabung des Instruments oder zumindest eines der vorhandenen Instrumente eingerichtet ist. Unter der Handhabung ist hier zu verstehen:
a) das Positionieren und/oder Bewegen des Instruments relativ zum biologischen Gewebe bzw. zum Patienten; und/oder
b) das Ausüben einer Kraft zwischen dem Instrument und dem zu behandelnden biologischen Gewebe aufweisen, beispielsweise wenn mittels des Instruments eine Klemmkraft und/oder eine Andrückkraft und/oder eine Schneidkraft auf das zu behandelnde biologische Gewebe ausgeübt werden soll.

Zum Halten des Instruments oder des wenigstens einen Instruments weist der Roboter eine Halteeinrichtung auf. Die Halteeinrichtung ist an einem bewegbaren Roboterarm des Roboters angeordnet.

Die Handhabung ist durch wenigstens einen Handhabungsparameter charakterisiert. Der wenigstens eine Handhabungsparameter ist im Roboter vorgegeben oder wird im Roboter ermittelt, beispielsweise in der Robotersteuerung oder mittels der Robotersteuerung.

Zur Ermittlung des wenigstens einen Handhabungsparameters sind außerhalb des Roboters keine zusätzlichen Sensoren am Instrument vorhanden, das mittels des Roboters gehalten wird. Unter solchen zusätzlichen Sensoren sind insbesondere Sensoren zu verstehen, die im Roboter für die Bewegungssteuerung des Roboterarms nicht ohnehin vorhanden sind und verwendet werden, sondern ausschließlich einem am Roboter angeordneten Instrument zugeordnet sind.

Der wenigstens eine Handhabungsparameter umfasst keine Betriebsparameter für den Betrieb des wenigstens einen an der Halteeinrichtung angeordneten Instruments. Der Handhabungsparameter oder zumindest einer der Handhabungsparameter oder alle Handhabungsparameter kann bzw. können lediglich unter Verwendung von elektrischen Steuerparametern und/oder elektrischen Robotersensorwerten ermittelt werden, die zur Steuerung des Roboters und insbesondere einer Antriebseinheit des Roboterarms ohnehin verwendet werden und somit verfügbar sind. Solche elektrischen Steuerparameter und/oder Robotersensorwerte können zur Steuerung oder Regelung einer insbesondere elektromotorischen Antriebseinheit des bewegbaren Roboterarms im Roboter verwendet beziehungsweise verfügbar sein. Jeder Handhabungsparameter kann dabei beispielsweise ein vorgegebener Sollwert für eine Antriebseinheit oder ein ermittelter bzw. gemessener Istwert einer Antriebseinheit sein, der die Position, die Bewegung oder die ausgeübte Kraft an der Antriebseinheit des Roboterarms beschreibt.

Das Chirurgiesystem hat außerdem eine Kommunikationseinrichtung. Die Kommunikationseinrichtung ist dazu eingerichtet, den wenigstens einen Handhabungsparameter des Roboters für die Gerätesteuerung bereitzustellen. Basierend auf dem wenigstens einen Handhabungsparameter ermittelt die Gerätesteuerung den wenigstens einen Betriebsparameter für das betreffende Instrument (z. B. das aktuell verwendete Instrument), zu dem der wenigstens eine Handhabungsparameter gehört, zum Beispiel während einer Behandlung von biologischem Gewebe. Basierend auf dem wenigstens einen Betriebsparameter wird dann das betreffende Instrument über das Versorgungsgerät versorgt. Abhängig von der Art des Betriebsparameters kann das Versorgungsgerät hierzu beispielsweise einen Generator zur Erzeugung einer Generatorspannung und/oder eines Generatorstroms aufweisen und/oder es kann eine Fluidquelle und/oder eine Lichtquelle und/oder eine Ultraschallquelle aufweisen. Der Generator und/oder die vorhandenen zur Versorgung dienenden Quellen können mittels der Gerätesteuerung gesteuert oder geregelt werden, um den wenigstens einen Betriebsparameter für das Instrument einzustellen.

Beispielsweise kann einer oder es können mehrere der nachfolgend genannten Parameter in beliebiger Kombination als Betriebsparameter verwendet werden:
- eine elektrische Leistung für das Instrument, insbesondere für eine Elektrode des Instruments;
- eine Aktivierungszeitdauer, während der kontinuierlich oder getaktet eine elektrische Leistung und/oder eine elektrische Spannung und/oder ein elektrischer Strom für das Instrument, insbesondere für eine Elektrode des Instruments, bereitgestellt wird;
- ein Stromparameter eines elektrischen Stroms für das Instrument, insbesondere für eine Elektrode des Instruments, wobei der Stromparameter eine Amplitude, eine Frequenz, ein Scheitelfaktor ("crest factor"), ein Tastgrad oder Aussteuergrad ("duty cycle"), eine Wellenform oder eine beliebige Kombination dieser Stromparameter sein kann;
- eine Spannungsparameter einer elektrischen Spannung für das Instrument, insbesondere für eine Elektrode des Instruments, wobei der Spannungsparameter eine Amplitude, eine Frequenz, ein Scheitelfaktor ("crest factor"), ein Tastgrad oder Aussteuergrad ("duty cycle"), eine Wellenform oder eine beliebige Kombination dieser Spannungsparameter sein kann;
- ein Fluidparameter eines zum Instrument geförderten Fluids, beispielsweise ein Druck, eine Strömungsgeschwindigkeit, ein Volumenstrom, ein Massenstrom oder eine beliebige Kombination dieser Fluidparameter;
- ein Ultraschallparameter einer dem Instrument bereitgestellten Ultraschallwelle, z. B. deren Frequenz und/oder Amplitude und/oder Leistung;
- ein Lichtparameter von dem Instrument bereitgestellten Licht, z. B. dessen Frequenz und/oder Amplitude und/oder Leistung.

Die erfindungsgemäße Ausgestaltung ermöglicht das schnelle und adaptive Einstellen des wenigstens einen Betriebsparameters für den Betrieb des wenigstens einen Instruments basierend auf aktuellen Informationen über die Handhabung des Instruments mittels des Roboters (charakterisiert durch den wenigstens einen Handhabungsparameter). Dadurch ist es der Gerätesteuerung bekannt, wie der Roboter das betreffende Instrument aktuell positioniert und/oder bewegt und/oder ob eine Kraft zwischen dem Instrument und dem zu behandelnden Gewebe auftritt und/oder wie groß eine solche Kraft ist (beispielsweise Klemmkraft, Andrückkraft, Schneidkraft).

Die Gerätesteuerung ist in der Lage, den wenigstens einen (z.B. elektrischen und/oder fluidischen) Betriebsparameter anzupassen, wenn dies aufgrund einer Veränderung eines Handhabungsparameters erforderlich sein sollte, beispielsweise wenn sich eine Kraft oder eine Bewegung des Instruments gegenüber dem zu behandelnden Gewebe ändert. Solche Anpassungen können im Versorgungsgerät bzw. der Gerätesteuerung sehr schnell und quasi in Echtzeit und zumindest ohne relevante Latenz ermittelt und eingestellt werden. Dadurch ist sichergestellt, dass der wenigstens eine Betriebsparameter zu jedem Zeitpunkt mit hoher Genauigkeit an die vom wenigstens einen Handhabungsparameter beschriebene Handhabung des Instruments angepasst ist.

Wie erläutert, sind zur Ermittlung des wenigstens einen Handhabungsparameters keine zusätzlichen Sensoren am Instrument erforderlich. Vielmehr werden dazu Sollwerte, Istwerte oder anderen Variable verwendet, die im Roboter bzw. der Robotersteuerung ohnehin vorliegen, um den Roboterarm zu positionieren und/oder zu bewegen und/oder mittels des Roboterarms eine gewünschte Kraft zwischen einem daran gehaltenen Instrument und einem zu behandelnden Gewebe auszuüben. Dadurch können zusätzlicher konstruktiver Aufwand wie auch Zusatzkosten vermieden werden. Außerdem wird der im Bereich des Instruments benötigte Bauraum nicht durch zusätzliche Sensoren vergrößert, was vorteilhaft für die Positionierung und/oder Bewegung des Instruments und dadurch wiederum für die Behandlung von biologischem Gewebe ist.

Bei einem Ausführungsbeispiel kann das Chirurgiesystem eine Bedieneinrichtung aufweisen, die als Mensch-Maschine-Schnittstelle bzw. Benutzerschnittstelle dient. Eine solche Bedieneinrichtung kann zur Eingabe und/oder Ausgabe von Informationen und Daten durch eine Bedienperson und/ oder an eine Bedienperson eingerichtet sein. Hierzu können Eingabeeinrichtungen und/oder Ausgabeeinrichtungen vorhanden sein, beispielsweise ein berührungsempfindlicher Bildschirm, Tasten, ein Lautsprecher, ein berührungsempfindliches Feld zur Bedienung eines Zeigerinstruments in einem Bildschirm, ein Steuerhebel (Joystick) oder andere beliebige Eingabeeinrichtungen und/oder Ausgabeeinrichtungen, wie sie an sich bekannt sind, beispielsweise aus dem Bereich der Maschinen- oder Robotersteuerung.

Die Kommunikationseinrichtung kann wenigstens eine Schnittstelle aufweisen oder durch wenigstens eine Schnittstelle gebildet sein. Beispielsweise kann die wenigstens eine Kommunikationsschnittstelle der Kommunikationseinrichtung Bestandteil der Gerätesteuerung und/oder Bestandteil der Robotersteuerung und/oder Bestandteil der Bedieneinrichtung sein.

Die Kommunikationseinrichtung kann Daten und Informationen drahtlos und/oder drahtgebunden übermitteln und kann hierzu die erforderlichen drahtlosen und/oder drahtgebundenen Kommunikationsschnittstellen aufweisen. Die Kommunikationseinrichtung ist insbesondere dazu eingerichtet, Daten und Informationen unidirektional oder bidirektional zwischen folgenden Teilnehmern zu übertragen: (i) zwischen der Robotersteuerung und der Gerätesteuerung und optional (ii) zwischen der Bedieneinrichtung und der Gerätesteuerung und optional (iii) zwischen der Bedieneinrichtung und der Robotersteuerung.

Die Kommunikationseinrichtung beziehungsweise die wenigstens eine Kommunikationsschnittstelle kann dazu eingerichtet sein, nach einem standardisierten Kommunikationsprotokoll zu arbeiten. Beispielsweise kann die wenigstens eine Kommunikationsschnittstelle eine USB-Schnittstelle, Ethernet-Schnittstelle, CAN-Schnittstelle, Bluetooth-Schnittstelle, WLAN-Schnittstelle, 5G-Schnittstelle, WiFi-Schnittstelle oder eine beliebige Kombination davon sein. Die Kommunikation kann bei einem Ausführungsbeispiel entsprechend dem Kommunikationsprotokoll IEEE 11073 SDC ("Service-oriented Device Connectivity") erfolgen.

Es ist vorteilhaft, wenn der Roboter zur Positionierung und/oder Bewegung der am Roboterarm angeordneten Halteeinrichtung wenigstens eine Antriebseinheit aufweist. Die wenigstens eine Antriebseinheit kann insbesondere dazu eingerichtet sein, zwei beweglich miteinander verbundene Armteile des Roboterarms relativ zueinander zu positionieren und/oder zu bewegen. Beispielsweise können solche Armteile relativ zueinander um wenigstens eine Achse gedreht bzw. relativ zueinander geschwenkt werden. Jede Antriebseinheit kann hierzu einen Elektromotor aufweisen, dessen Betrieb die Relativbewegung zwischen den beiden Armteilen durchführt. Jede Antriebseinheit kann durch die Robotersteuerung individuell gesteuert werden, so dass eine Relativbewegung zwischen zwei unmittelbar verbundenen Armteilen des Roboterarms unabhängig von einer Relativbewegung zweier anderer unmittelbar miteinander verbundener Armteile durchgeführt werden kann.

Der Betrieb eines Elektromotors kann durch wenigstens einen Motorparameter beschrieben werden. Ein Motorparameter kann ein vorgegebener Sollwert, ein gemessener Istwert oder ein anderweitig ermittelter Istwert sein. Bei dem Motorparameter kann es sich beispielsweise um elektrische Parameter handeln, wie zum Beispiel eine Motorspannung, ein Motorstrom, eine elektrische Leistung des Motors, eine Frequenz der Motorspannung und/oder des Motorstromes oder ähnliches. Zusätzlich oder alternativ kann der Motorparameter ein Drehmoment des Elektromotors und/oder eine Drehzahl des Elektromotors und/oder ein Drehwinkel des Elektromotors und/oder eine zeitliche Änderung irgendeines vorstehend angegebenen Motorparameters sein (zeitliche Ableitung einer beliebigen Ordnung, beispielsweise erster Ordnung oder zweiter Ordnung).

Der Motorparameter, einer der Motorparameter oder alle Motorparameter kann bzw. können beispielsweise sensorlos ermittelt werden. Optional kann zumindest eine der vorhandenen Antriebseinheiten wenigstens einen Motorsensor aufweisen, beispielsweise um eine Motordrehzahl und/oder ein Motordrehmoment sensorisch zu ermitteln. Ein solcher Motorsensor stellt einen Robotersensor des Roboters und keinen zusätzlichen Sensor dar. Abgesehen von derartigen Motorsensoren kann der Roboter auf weitere Sensoren verzichten. Der wenigstens eine, optional vorhandene Robotersensor ist vorzugsweise ein zur Detektion einer Relativbewegung und/oder einer Relativposition eingerichteter Sensor.

Ein Handhabungsparameter kann unmittelbar ein Sollwert und/oder Istwert sein, beispielsweise einer der vorstehend erläuterten Motorparameter, der im Roboter verfügbar ist. Zusätzlich oder alternativ können basierend auf wenigstens einem Sollwert und/oder Istwert bzw. Motorparameter durch Verwenden von einer Funktion, einer Kennlinie, einem Kennfeld, einer Tabelle ("look-up table") wenigstens ein Handhabungsparameter ermittelt werden.

Bei einer bevorzugten Ausführungsform kann der über die Gerätesteuerung gesteuerte oder geregelte Betriebsparameter für das Instrument oder für eines der an das Versorgungsgerät angeschlossenen Instrumente abhängig von einer Bewegungsgeschwindigkeit des betreffenden Instruments relativ zu dem zu behandelnden Gewebe ermittelt und eingestellt werden. Hierzu kann beispielsweise eine elektrische Leistung und/oder ein elektrischer Strom und/oder eine elektrische Spannung an die Bewegungsgeschwindigkeit angepasst werden.

Bei einem Ausführungsbeispiel wird abhängig von der Bewegungsgeschwindigkeit des Instruments die elektrische Leistung am Instrument bzw. an wenigstens einer Elektrode des Instruments verändert und dabei insbesondere erhöht, wenn die Bewegungsgeschwindigkeit zunimmt. Die Leistung kann in einer oder mehreren Stufen oder kontinuierlich abhängig von der Geschwindigkeit verändert werden. Es ist dabei insbesondere vorteilhaft, wenn die elektrische Leistung am Instrument verändert und die elektrische Spannung an der wenigstens einen Elektrode des Instruments dabei konstant gehalten wird.

Bei irgendeinem Ausführungsbeispiel kann optional die Bewegung bzw. Position des Roboterarms basierend auf wenigstens einem ermittelten Betriebsparameter und/oder Behandlungsparameter und/oder Gewebeparameter des zu behandelndes Gewebes gesteuert oder geregelt werden. Beispielsweise kann der Roboterarm in einer Position angehalten werden oder eine definierte Bewegung ausführen, wenn das Instrument eine Position erreicht hat, in der ein bestimmter Gewebetyp erkannt wurde, beispielsweise Tumorgewebe. Die Erkennung kann basierend auf wenigstens einem ermittelten Parameter erfolgen, beispielsweise einer Gewebeimpedanz und/oder basierend auf einem Spektrum des Gewebes (Optische Emissionsspektroskopie, OES). Die Bewegung des Roboterarm kann zusätzlich oder alternativ auch abhängig davon erfolgen, ob ein vorgegebener Behandlungseffekt erreicht wurde.

Es kann insbesondere vorteilhaft sein, eine am Instrument und insbesondere an wenigstens einer Elektrode des Instruments bereitgestellte elektrische Leistung abzuschalten, wenn das Instrument relativ zum behandelten Gewebe stillsteht oder wenn seit dem Beginn des Stillstands des Instruments relativ zum behandelten Gewebe eine vorgegebene minimale Stillstandsdauer abgelaufen ist.

Zusätzlich oder alternativ kann der wenigstens eine Betriebsparameter auch abhängig von einer Kraft zwischen dem betreffenden Instrument und dem zu behandelnden Gewebe (z.B. Schneidkraft und/oder Andrückkraft und/oder Klemmkraft) verändert werden. Die Veränderung kann stufenweise oder kontinuierlich erfolgen. Beispielsweise kann abhängig von einem die Kraft beschreibenden Handhabungsparameter ein Betriebsparameter oder es können mehrere Betriebsparameter verändert werden. Bei einem Ausführungsbeispiel kann wenigstens ein Betriebsparameter mit zunehmender Kraft wie folgt verändert werden:
- die elektrische Leistung am Instrument wird kontinuierlich oder stufenweise mit zunehmender Kraft erhöht;
- die elektrische Spannung am Instrument wird stufenweise oder kontinuierlich mit zunehmender Kraft erhöht;
- der Scheitelfaktor wird stufenweise oder kontinuierlich mit zunehmender Kraft verringert.
Alle anderen elektrischen Betriebsparameter können dabei unabhängig von der Kraft eingestellt uns beispielsweise konstant gehalten werden.

Der wenigstens eine Betriebsparameter, insbesondere zumindest ein elektrischer Betriebsparameter, kann abhängig von einer Eindringtiefe oder einer Schnitttiefe eines Werkzeugs eines (beispielsweise elektrochirurgischen) Instruments in das zu behandelnde Gewebe verändert werden, insbesondere wie folgt:
- die elektrische Leistung am elektrochirurgischen Instrument kann mit zunehmender Eindringtiefe oder Schnitttiefe stufenweise oder kontinuierlich erhöht werden;
- der Scheitelfaktor kann mit zunehmender Eindringtiefe oder Schnitttiefe stufenweise oder kontinuierlich verringert werden.
Alle anderen elektrischen Betriebsparameter können dabei unabhängig von der Eindringtiefe bzw. Schnitttiefe eingestellt und beispielsweise konstant gehalten werden.

Wenn das Instrument zwei relativ zueinander bewegbare und insbesondere relativ zueinander schwenkbare Instrumententeile aufweist, kann die Relativposition (zum Beispiel ein Relativwinkel) und/oder eine Relativbewegung und/oder eine Kraft zwischen den beiden bewegbaren Instrumententeilen als wenigstens ein Handhabungsparameter ermittelt und abhängig davon wenigstens ein Betriebsparameter verändert werden. Solche Instrumententeile können beispielsweise relativ zueinander schwenkbare Branchen eines zangenartigen Werkzeugs bzw. Instruments und/oder ein in einem Instrumentenkanal verschiebbares Werkzeug (z.B. Nadel oder Messer) sein.

Beispielsweise kann Gewebe zwischen den Instrumententeilen bzw. Branchen eingeklemmt werden, um das Gewebe zu koagulieren und optional zusätzlich zu durchtrennen. Vorzugsweise kann dabei der Betriebsparameter abhängig von einer ermittelten Klemmkraft oder Schließkraft zwischen zwei beweglichen Instrumententeilen wie folgt verändert werden:
- eine elektrische Leistung an dem wenigstens einen bewegbaren Werkzeugteil wird mit zunehmender Kraft zwischen den beiden Werkzeugteilen stufenweise oder kontinuierlich verringert;
- eine Aktivierungszeitdauer, während der an wenigstens einem der bewegbaren Werkzeugteile eine elektrische Leistung bereitgestellt wird, wird mit zunehmender Kraft zwischen den beiden Werkzeugteilen kontinuierlich oder stufenweise verringert.
Alle anderen elektrischen Betriebsparameter können dabei unabhängig von der Klemmkraft oder Schließkraft zwischen zwei beweglichen Instrumententeilen eingestellt uns beispielsweise konstant gehalten werden.

Abhängig von der Relativposition oder einem Relativwinkel zwischen den beiden bewegbaren Werkzeugteilen kann wenigstens ein Betriebsparameter wie folgt verändert werden:
- eine elektrische Leistung an wenigstens einem der bewegbaren Werkzeugteile kann mit größer werdendem Relativabstand oder größer werdendem Winkel zwischen den Werkzeugteilen stufenweise oder kontinuierlich erhöht werden;
- eine Aktivierungszeitdauer, während der eine elektrische Leistung an wenigstens einem der bewegbaren Instrumententeile anliegt, kann mit zunehmendem Relativabstand oder zunehmendem Winkel zwischen den bewegbaren Werkzeugteilen stufenweise oder kontinuierlich erhöht werden.
Alle anderen elektrischen Betriebsparameter können dabei unabhängig von der Relativposition bzw. dem Relativwinkel zwischen den beiden bewegbaren Werkzeugteilen eingestellt uns beispielsweise konstant gehalten werden.

Zur Ermittlung einer Kontakt- oder Referenzposition relativ zu dem zu behandelnden Gewebe kann der Zeitpunkt einer Berührung oder eines Anlagekontakts zwischen dem Instrumententeil und dem zu behandelnden Gewebe ermittelt werden, beispielsweise mittels der Gerätesteuerung und/oder mittels der Robotersteuerung. Wenn an einem Instrumententeil eine Spannung anliegt, kann eine Spannungsänderung beim Kontakt des Instrumententeils mit zu behandelndem Gewebe mittels der Gerätesteuerung erkannt werden. Zusätzlich oder alternativ kann bei einem solchen Kontakt eine Stromänderung eines in das Instrument bzw. das Instrumententeil fließenden Stromes erkannt werden. Wenn das zu behandelnde Gewebe einen ausreichend großen Widerstand gegen das Eindringen eines Instrumententeils entgegenbringt, kann zusätzlich oder alternativ auch ein ansteigender Motorstrom in einer Antriebseinheit des Roboters ermittelt werden, der erforderlich ist, um die zunehmende Gegenkraft zu überwinden.

Diese Kontakt- oder Referenzposition des an der Halteeinrichtung angeordneten Instruments mit dem Gewebe kann zur Ermittlung einer Eindringtiefe bzw. Schnitttiefe in das zu behandelnde Gewebe verwendet werden, wobei kein zusätzlicher Sensor erforderlich ist. Eine Positionsänderung gegenüber dieser Kontakt- oder Referenzposition in das zu behandelnde Gewebe entspricht der Eindringtiefe bzw. Schnitttiefe.

Der Roboter und beispielsweise die Halteeinrichtung kann dazu eingerichtet sein, zwei relativ zueinander bewegliche und beispielsweise relativ zueinander schwenkbewegliche Instrumententeile eines an der Halteeinrichtung angeordneten Instruments relativ zueinander zu bewegen. Die Relativposition und/oder Relativbewegung und/oder Kraft zwischen den beweglichen Werkzeugteilen kann durch den Roboter und insbesondere mittels der Robotersteuerung ermittelt werden. Beispielsweise können die relativ zueinander beweglichen Instrumententeile in eine Referenzposition (zum Beispiel vollständig geöffnete Position oder vollständig geschlossene, unmittelbar aneinander anliegende Position) bewegt werden. Im Anschluss an diese Referenzposition durchgeführte Positionsänderungen können ermittelt werden, um die aktuelle Relativposition und die Relativbewegung (zum Beispiel Relativgeschwindigkeit) zu ermitteln ohne einen zusätzlichen Sensor zu benötigen.

Eine zur Betätigung der beweglichen Instrumententeile mittels des Roboters bzw. der Halteeinrichtung aufzubringende Kraft kann gemessen und/oder anderweitig im Roboter ermittelt werden. Beispielsweise kann ein zur Betätigung erforderlicher Motorstrom in einer Antriebseinheit charakteristisch sein für die zwischen den bewegbaren Instrumententeilen ausgeübte Schließkraft bzw. Klemmkraft.

Vorteilhafte Ausgestaltungen der Erfindung ergeben sich aus den abhängigen Ansprüchen, der Beschreibung und der Zeichnung. Nachfolgend werden bevorzugte Ausführungsbeispiele basierend auf der beigefügten Zeichnung im Einzelnen erläutert. In der Zeichnung zeigen:
Figur 1 eine schematische, blockschaltbildähnliche Prinzipdarstellung eines Chirurgiesystems aufweisend einen Roboter sowie eine Vorrichtung,
Figur 2 ein Blockschaltbild eines Ausführungsbeispiels einer Vorrichtung für das Chirurgiesystem nach Figur 1,
Figur 3 ein Blockschaltbild eines Ausführungsbeispiels mit mehreren Antriebseinheiten des Roboters aus Figur 1 die mittels einer Robotersteuerung gesteuert werden und

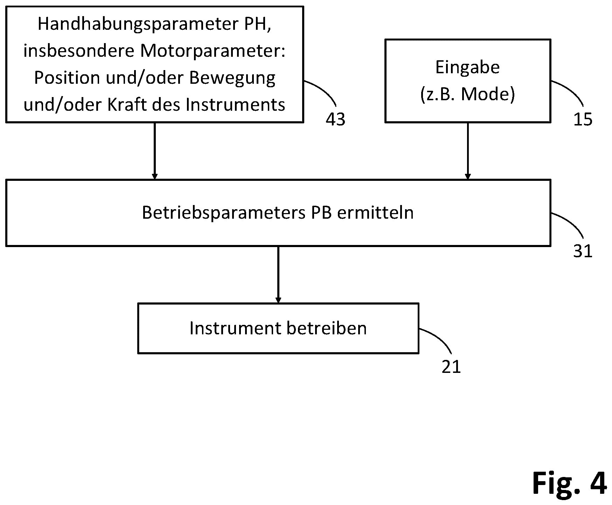

Figur 4 eine schematische Prinzipdarstellung nach Art eines Blockschaltbilds zur Darstellung eines Zusammenhangs zwischen der Steuerung des Roboters und der abhängig davon durchgeführten Steuerung der Vorrichtung.

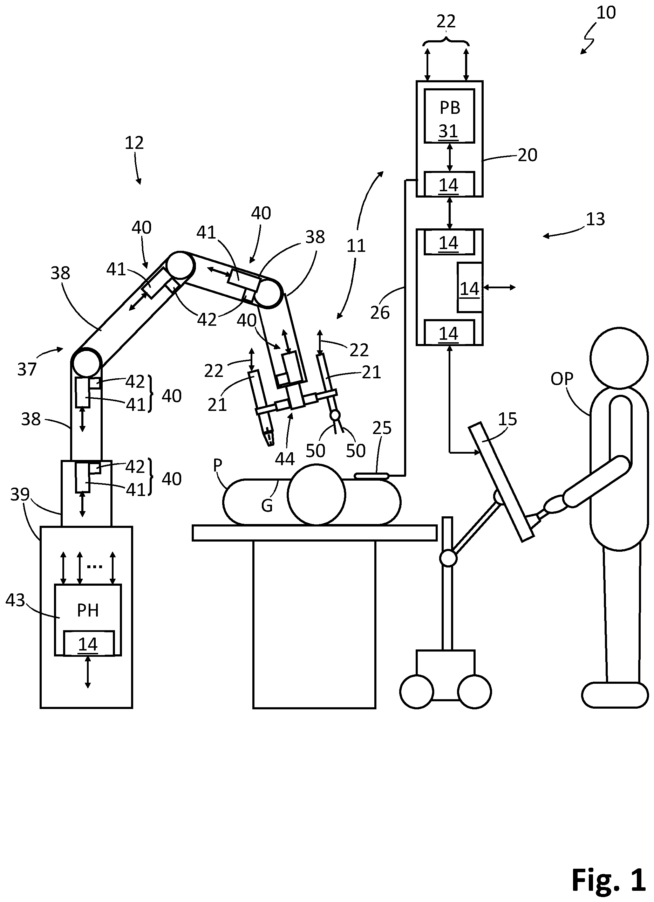

In Figur 1 ist nach Art eines Blockschaltbilds ein Ausführungsbeispiel eines Chirurgiesystems 10 stark schematisiert dargestellt. Das Chirurgiesystem 10 weist eine Vorrichtung 11 und einen Roboter 12 auf. Die Vorrichtung 11 und der Roboter 12 sind mittels einer Kommunikationseinrichtung 13 kommunikationsverbunden, um elektrische und/oder optische Signale übertragen zu können, vorzugsweise bidirektional.

Das Chirurgiesystem 10 hat beim Ausführungsbeispiel außerdem eine Bedieneinrichtung 15, mittels der das Chirurgiesystem 10 durch eine Bedienperson OP bedient werden kann. Die Bedieneinrichtung 15 stellt somit eine Bedienschnittstelle oder Mensch-Maschine-Schnittstelle dar. Die Bedieneinrichtung kann über geeignete Eingabeeinheiten und/oder Ausgabeeinheiten zum Eingeben von Befehlen und/ oder zum Ausgeben von Informationen eingerichtet sein. Die Bedieneinrichtung 15 kann beispielsweise einen Bildschirm, ein berührungsempfindliches Feld bzw. Bildschirm, Bewegungssteuerelemente oder ähnliches aufweisen.

Über die Bedieneinrichtung 15 kann der Roboter 12 und vorzugsweise auch die Vorrichtung 11 von der Bedienperson OP bedient und gesteuert werden. Eine Bedienperson kann beispielsweise eine Anwendung (Mode) für die Vorrichtung 11 über die Bedieneinrichtung 15 und/oder unmittelbar an der Vorrichtung 11 auswählen oder einstellen. Eine Anwendung kann beispielsweise die Behandlungsart (z.B. Schneiden, Koagulieren, Abladieren, etc.) von biologischen Gewebe G eines Patienten P sein.

Die Kommunikationseinrichtung 13 kann zur Herstellung der Kommunikationsverbindung wenigstens eine Kommunikationsschnittstelle 14 aufweisen, wobei jede Kommunikationsschnittstelle 14 eine drahtgebundene und/oder drahtlose Kommunikationsschnittstelle sein kann. Die Kommunikation kann basierend auf einem standardisierten Kommunikationsprotokoll erfolgen. Beispielsweise kann die Kommunikationsschnittstelle eine WLAN-Schnittstelle, eine 5G-Schnittstelle, eine USB-Schnittstelle, eine Bluetooth-Schnittstelle, eine WIFI-Schnittstelle, irgendeine andere standardisierte Schnittstelle oder eine beliebige Kombination hiervon sein.

Die Vorrichtung 11 hat ein Versorgungsgerät 20, an das ein mittels des Versorgungsgeräts 20 betriebenes Instrument 21 angeschlossen ist oder angeschlossen werden kann. Optional können an das Versorgungsgerät 20 auch mehrere Instrumente 21 angeschlossen sein.

Bei dem hier veranschaulichten Ausführungsbeispiel werden beispielhaft elektrochirurgische Instrumente 21 beschrieben. Die Vorrichtung 11 kann daher als elektrochirurgische Vorrichtung bezeichnet werden. Zusätzlich oder alternativ sind auch andere Instrumente 21 verwendbar, beispielsweise Ultraschallinstrumente oder Kryoinstrumente. Abhängig vom Instrumententyp wird vom Versorgungsgerät 20 der wenigstens eine Betriebsmedium bereitgestellt, also beispielsweise eine elektrische Leistung und/oder ein Fluid und/oder Ultraschall, etc.

Zur Verbindung mit dem Versorgungsgerät 20 hat das Instrument 21 eine Leitung 22. Über diese Leitung 22 kann eine elektrische und/oder optische und/oder fluidische und/oder Ultraschall leitende Verbindung mit dem Versorgungsgerät 20 hergestellt werden. Dazu weist die Leitung 22 an ihrem dem Instrument 21 entgegengesetzten Ende einen Stecker 23 auf, mittels dem eine lösbare Verbindung mit einer Buchse oder einer anderen geeigneten Anschlusseinrichtung des Versorgungsgeräts 20 hergestellt werden kann.

Das elektrochirurgische Instrument 21 kann beispielsweise als monopolares Instrument, bipolares Instrument oder multipolares Instrument ausgebildet sein. Es weist dann wenigstens eine mit einer elektrischen Spannung und/oder einem elektrischen Strom betreibbare Instrumentenelektrode 24 auf.

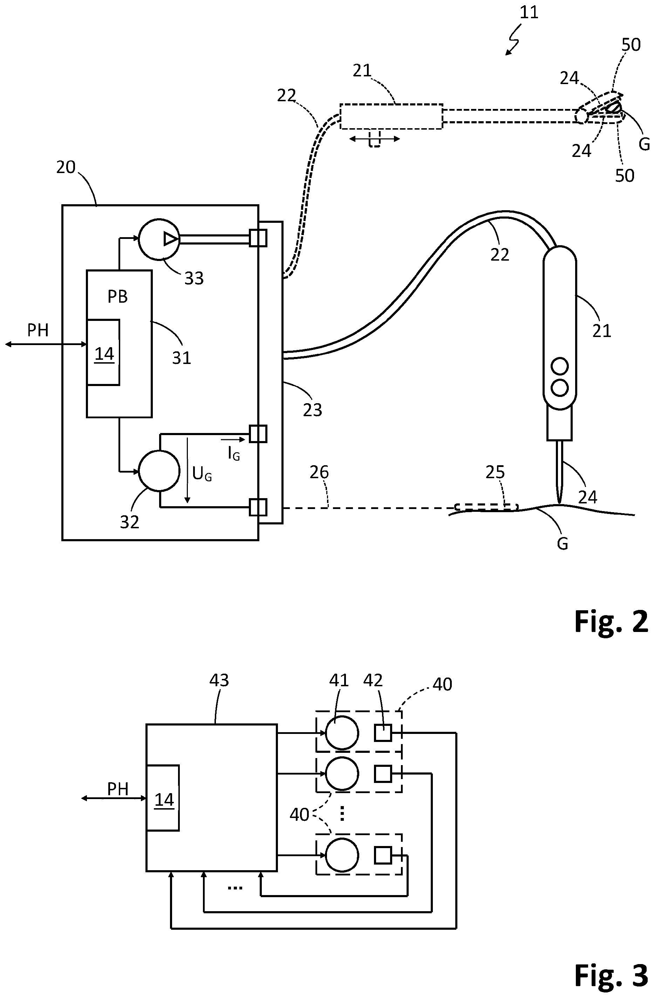

Bei einem monopolaren Instrument beziehungsweise einem als monopolares Instrument betriebenen bipolaren oder multipolaren Instrument weist die elektrochirurgische Vorrichtung zusätzlich eine Neutralelektrode 25 auf, die bei bipolar oder multipolar arbeitenden Instrumenten entfallen kann. Die Neutralelektrode 25 wird zur Behandlung von biologischem Gewebe G eines Patienten P elektrisch leitend am Patienten P angebracht (Figur 1). Dabei kann ein Behandlungsstromkreis vom Versorgungsgerät 20, über die Instrumentenelektrode 25, das zu behandelnde Gewebe G des Patienten P und die Neutralelektrode 25 mit dem Versorgungsgerät 20 geschlossen werden (Figuren 1 und 2). Hierzu ist die Neutralelektrode 25 über eine Elektrodenleitung 26 elektrisch mit dem Versorgungsgerät 20 verbunden.

Im Falle eines bipolaren oder multipolaren Instruments 21 (gestrichelt in Figur 2 dargestellt) kann der Behandlungsstromkreis vom Versorgungsgerät 20, über eine der vorhandenen Instrumentenelektroden, das zu behandelnde Gewebe G des Patienten P, eine andere der vorhandenen Instrumentenelektroden mit dem Versorgungsgerät 20 geschlossen werden (ohne Neutralelektrode 25). Die Darstellung in Figur 2 ist schematisch. Es versteht sich, dass jedes Instrument 21 über einen individuellen Stecker 23 mit dem Versorgungsgerät 20 verbunden werden kann.

Das Versorgungsgerät 20 hat zur Versorgung des Instruments 21 mit elektrischer Energie einen mittels einer Gerätesteuerung 31 steuerbaren elektrischen Generator 32. Über den Generator 32 kann das Instrument mit einer elektrischen Leistung versorgt werden, die beispielsweise an die wenigstens eine Instrumentenelektrode 24 angelegt werden kann. Der Generator 32 kann als eingeprägte Spannungsquelle oder als eingeprägte Stromquelle arbeiten. Über den Generator 32 kann eine hochfrequente Generatorspannung U_{G} und/oder ein hochfrequenter Generatorstrom I_{G} für das angeschlossene Instrument 21 bereitgestellt werden.

Das Versorgungsgerät 20 kann optional eine mittels der Gerätesteuerung 31 steuerbare Fluidquelle 33 aufweisen. Dadurch besteht die Möglichkeit, ein angeschlossenes elektrochirurgisches Instrument 21 für den Betrieb mit einem Fluid und insbesondere einem Gas zu versorgen, beispielsweise Argon. Das Gas kann beispielsweise im elektrochirurgischen Instrument 21 zur Erzeugung eines Plasmas für die Gewebebehandlung verwendet werden. Wie erwähnt können auch andere Instrumententypen verwendet werden.

Der Roboter 12 hat einen bewegbaren bzw. positionierbaren Roboterarm 37. Wie es in der schematischen Darstellung nach Figur 1 zu erkennen ist, weist der Roboterarm 37 mehrere relativ zueinander bewegbare Armteile 38 auf. Zwei unmittelbar benachbarte Armteile 38 sind beispielsgemäß schwenkbar aneinander gelagert. Der Roboterarm 37 bzw. einer der Armteile 38 kann drehbar und/oder schwenkbar an einer Basis 39 des Roboters 12 angeordnet sein, wobei sich die Drehachse beispielsgemäß in Vertikalrichtung erstrecken kann.

Zur Drehung und/oder Durchführung einer Schwenkbewegung oder einer anderen Bewegung zwischen dem Roboterarm 37 und der Basis 39 bzw. zwischen zwei bewegbar miteinander verbundenen Armteilen 38 weist der Roboter 12 mehrere Antriebseinheiten 40 auf. Die Antriebseinheiten 40 können hierzu jeweils einen steuerbaren Elektromotor 41 aufweisen. Die Antriebseinheiten 40 können optional einen dem Elektromotor 41 zugeordneten Motorsensor 42 aufweisen, beispielsweise einen Drehlagen- und/oder Drehzahlsensor.

Der Roboter 12 weist eine Robotersteuerung 43 auf. Die Robotersteuerung 43 ist dazu eingerichtet, die Antriebseinheiten 40 des Roboterarms 37 zu steuern. Dadurch können die Armteile 38 relativ zueinander und/oder der Roboterarm 37 relativ zur Basis 39 bewegt und positioniert werden. Der Robotersteuerung 43 können optional die Signale der Motorsensoren 42 zur Steuerung der jeweiligen Antriebseinheit 40 übermittelt werden, wenn die betreffende Antriebseinheit 40 über einen Motorsensor 42 verfügt. Zusätzlich oder alternativ können Motorparameter in der Robotersteuerung 43 vorgegeben und/oder ermittelt werden, zum Beispiel eine Motorspannung und/oder ein Motorstrom und/oder ein Motordrehmoment und/oder eine Motordrehzahl und/oder eine zeitliche Änderung irgendeines der vorstehend genannten Motorparameter der jeweiligen Antriebseinheit 40.

An dem der Basis 39 entgegengesetzten Ende weist der Roboterarm 37 eine Halteeinrichtung 44 auf, die zum Halten bzw. Anordnen eines Instruments oder optional auch mehrerer Instrumente 21 eingerichtet ist. Die Halteeinrichtung 44 kann beispielsweise als Greifeinrichtung ausgeführt sein.

Mittels des Roboterarms 37 kann die Halteeinrichtung 44 und somit ein dort gehaltenes Instrument 21 relativ zum Patienten P bewegt und positioniert werden, so dass das Instrument 21 relativ zu dem zu behandelnden Gewebe G ausgerichtet werden kann. Die Bewegung des Roboterarms 37 und damit des dort gehaltenen Instruments 21 kann eine Bedienperson OP beim Ausführungsbeispiel mittels der Bedieneinrichtung 15 steuern bzw. beeinflussen.

Ein Instrument 21 wird mittels des Roboters 12 gehandhabt, wobei Handhabungsparameter PH diese Handhabung steuern oder regeln oder beschreiben, Die Handhabung umfasst die Relativposition und/oder die Relativgeschwindigkeit zwischen einem Instrument 21 und zu behandelndem Gewebe G und/oder eine Kraft, die mittels des Instruments 21 auf das zu behandelnde Gewebe G ausgeübt wird. Der wenigstens eine Handhabungsparameter PH wird mittels der Robotersteuerung 43 vorgegeben oder ermittelt und kann beispielsweise wenigstens ein Sollwert und/oder wenigstens ein Istwert der Bewegung des Roboterarms sein oder davon abgeleitet werden. Der wenigstens eine Handhabungsparameter HP beschreibt insbesondere die Steuerung der Antriebseinheiten 40 und kann ein elektrischer Motorparameter des Elektromotors 41 sein.

Während die Robotersteuerung 43 den Roboterarm 37 entsprechend dem wenigstens einen Handhabungsparameter PH steuert oder regelt, kann mittels der Gerätesteuerung 31 wenigstens ein Betriebsparameter PB des betreffenden Instruments 21 gesteuert oder geregelt werden. Der Betriebsparameter PB ist ein das bereitgestellte Betriebsmedium beschreibender Parameter, beispielsweise ein elektrischer und/oder fluidischer Parameter, der den Betrieb des betreffenden elektrochirurgischen Instruments 21 beschreibt.

Als Betriebsparameter PB kann zum Beispiel wenigstens einer der nachfolgenden Parameter mittels der Gerätesteuerung 31 im Versorgungsgerät 20 gesteuert oder geregelt werden:
- ein Stromparameter des Generatorstroms IG, wobei der Stromparameter eine Amplitude, eine Frequenz, ein Scheitelfaktor ("crest factor"), ein Tastgrad oder Aussteuergrad ("duty cycle"), eine Wellenform oder eine beliebige Kombination dieser Stromparameter sein kann;
- eine Spannungsparameter der Generatorspannung UG, wobei der Spannungsparameter eine Amplitude, eine Frequenz, ein Scheitelfaktor ("crest factor"), ein Tastgrad oder Aussteuergrad ("duty cycle"), eine Wellenform oder eine beliebige Kombination dieser Spannungsparameter sein kann;
- ein Fluidparameter eines zum Instrument geförderten Fluids, beispielsweise ein Druck, eine Strömungsgeschwindigkeit, ein Volumenstrom, ein Massenstrom oder eine beliebige Kombination dieser Fluidparameter;
- eine Aktivierungszeitdauer, während der kontinuierlich oder getaktet eine Generatorspannung UG und/oder ein Generatorstrom IG und/oder eine elektrische Leistung für ein angeschlossenes Instrument 21 bereitgestellt wird;
- eine elektrische Leistung für das Instrument, insbesondere für eine Elektrode des Instruments.

Die Betriebsparameter PB unterscheiden sich von den Handhabungsparametern PH. Während die Betriebsparameter PB einen (beispielsweise elektrischen und/oder fluidischen) Betriebszustand eines angeschlossenen Instruments 21 definieren, geben die Handhabungsparameter PH die Position (räumliche Position und/oder Ausrichtung bzw. Orientierung im Raum) eines an der Halteeinrichtung 44 des Roboters 12 angeordneten Instruments 21 relativ zu dem zu behandelnden Gewebe G des Patienten P an. Zusätzlich kann ein Handhabungsparameter PH auch eine Kraft (z.B. Andrückkraft, Klemmkraft, Schnittkraft, Vorschubkraft) zwischen einem Instrument 21 und zu behandelndem Gewebe G beschreiben.

Mittels der Kommunikationseinrichtung 13 bzw. der wenigstens einen Kommunikationsschnittstelle 14 sind die Robotersteuerung 43 und die Gerätesteuerung 31 kommunikationsverbunden, wobei zumindest eine Übertragung von Kommunikationsdaten von der Robotersteuerung 43 zur Gerätesteuerung 31 ermöglicht wird. Die Kommunikationsverbindung kann auch bidirektional sein. Zusätzlich oder alternativ kann außerdem eine Kommunikationsverbindung zwischen der Bedieneinrichtung 15 einerseits und der Gerätesteuerung 31 und/oder der Robotersteuerung 43 andererseits über die Kommunikationseinrichtung 13 hergestellt werden, wie es schematisch in Figur 1 veranschaulicht ist.

Mittels der Kommunikationsverbindung bzw. der Kommunikationseinrichtung 13 wird der Gerätesteuerung 31, der wenigstens eine Handhabungsparameter PH oder zumindest einer der Handhabungsparameter PH bereitgestellt. Die Gerätesteuerung 31 ist dazu eingerichtet, basierend auf dem Handhabungsparameter PH oder zumindest einem der Handhabungsparameter PH, den Betriebsparameter PB oder zumindest einen der Betriebsparameter PB zu ermitteln. Zumindest einer der Betriebsparameter PB, mehrere oder alle Betriebsparameter PB werden somit abhängig von einem oder mehreren Handhabungsparametern PH ermittelt und gesteuert oder geregelt.

Beispielsweise können ein Betriebsparameter PB oder mehrere Betriebsparameter PB abhängig von der Relativgeschwindigkeit eines Instruments 21 relativ zum Gewebe G und/oder einer Kraft zwischen einem Instrument 21 und dem Gewebe G ermittelt und gesteuert oder geregelt werden. Dabei wird insbesondere die elektrische Spannung und/oder der elektrische Strom und/oder die elektrische Leistung an der wenigstens einen Instrumentenelektrode 24 geschwindigkeitsabhängig oder kraftabhängig ermittelt und dementsprechend gesteuert oder geregelt.

Konkret kann der elektrische Betrieb eines elektrochirurgischen Instruments 21 abhängig von seiner Relativgeschwindigkeit oder seiner Relativkraft gegenüber dem zu behandelnden Gewebe G gemäß einem oder mehreren der nachfolgend beschriebenen Möglichkeiten verändert bzw. angepasst werden. Die Veränderung bzw. Anpassung des Betriebsparameters PB bzw. von zumindest einem Betriebsparameter PB kann dabei jeweils stufenlos und/oder kontinuierlich positions- und/oder geschwindigkeits- und/oder kraftabhängig verändert werden. In der nachfolgenden Erläuterung bezieht sich die Relativgeschwindigkeit auf die Geschwindigkeit des elektrochirurgischen Instruments 21 relativ zu dem zu behandelnden Gewebe G und die Kraft auf eine zwischen dem elektrochirurgischen Instrument 21 und dem zu behandelnden Gewebe G ausgeübte Kraft (zum Beispiel Andrückkraft an das Gewebe G, Vorschubkraft beim Bewegen des Instruments entlang der Oberfläche des Gewebes G oder in das Gewebe G, Klemmkraft zwischen zwei beweglichen Instrumententeilen oder Schnittkraft beim Schneiden des Gewebes G):
1) Eine dem elektrochirurgischen Instrument 21 bzw. der wenigstens einen Instrumentenelektrode 24 bereitgestellte elektrische Leistung wird mit zunehmender Relativgeschwindigkeit erhöht, wobei die dem elektrochirurgischen Instrument 21 bereitgestellte elektrische Spannung (insbesondere Generatorspannung U_{G}) konstant gehalten werden kann.
2) Mit zunehmender Kraft kann die elektrische Leistung erhöht werden, die dem wenigstens einen elektrochirurgischen Instrument 21 und insbesondere der Instrumentenelektrode 24 bereitgestellt wird. Dabei kann beispielsweise die dem elektrochirurgischen Instrument 21 bereitgestellte Spannung (insbesondere Generatorspannung U_{G}) mit zunehmender Kraft erhöht werden. Vorzugsweise kann ein Scheitelfaktor der Generatorspannung U_{G} und/oder des Generatorstromes I_{G} mit zunehmender Kraft verringert werden.
3) Eine Aktivierungsdauer, während der eine elektrische Leistung für das elektrochirurgische Instrument 21 bzw. die Instrumentenelektrode 24 kontinuierlich oder getaktet bereitgestellt wird, kann mit zunehmender Kraft verringert werden, wobei die elektrische Leistung reduziert werden kann, insbesondere wenn die Kraft eine Klemmkraft beschreibt, mit der Gewebe G zwischen zwei beweglichen Teilen des Instruments geklemmt wird (vgl. Figur 2);
4) Mit zunehmendem Abstand von zwei beweglichen Instrumententeilen des Instruments, die das zu behandelnde Gewebe G beaufschlagen und beispielsweise klemmen (vgl. Figur 2), kann eine Aktivierungszeitdauer vergrößert werden, während der kontinuierlich oder getaktet eine elektrische Leistung für das elektrochirurgische Instrument 21 bereitgestellt wird, wobei insbesondere die elektrische Leistung mit zunehmendem Abstand erhöht werden kann.

Die beiden beweglichen Instrumententeile eines Instruments 21 können beispielsweise Branchen 50 eines elektrochirurgischen Instruments und insbesondere eines zangenartig arbeitenden elektrochirurgischen Instruments sein (beispielhaft in Figuren 1 und 2 gezeigt). Die Branchen 50 können aufeinander zu und voneinander weg bewegt und beispielsweise geschwenkt werden. Der Relativabstand ist hier durch den Winkel zwischen den Branchen beschrieben und ist groß, wenn der Winkel zwischen den Branchen groß ist, wohingegen der Relativabstand klein ist, wenn der Winkel zwischen den Branchen klein ist.

Die beweglichen Instrumententeile bzw. Branchen 50 eines an der Halteeinrichtung 44 angeordneten (beispielsweise elektrochirurgischen) Instruments 21 können mittels des Roboters 12 bzw. Mittels der Halteeinrichtung 44 betätigt werden, beispielsweise um zu behandelndes Gewebe G zwischen den Branchen 50 klemmend zu beaufschlagen. An den Branchen 50 kann jeweils eine Instrumentenelektrode 24 vorhanden sein, die an zu behandelndem Gewebe G anliegt, wenn es zwischen den Branchen 50 geklemmt wird. Dadurch kann Gewebe G beispielsweise koaguliert werden.

Durch die erfindungsgemäße Ausgestaltung kann ein Betriebsparameter PB oder es können mehrere Betriebsparameter PB schnell auf veränderte Handhabungsparameter PH angepasst werden. Dadurch kann die Behandlung von Gewebe G optimiert werden. Betriebsparameter, beispielsweise elektrische und/oder fluidische Betriebsparameter, können sehr schnell an veränderte Bewegungsgeschwindigkeiten, Kräfte oder andere Handhabungsparameter PH angepasst werden, die der Roboter 12 auf das Instrument 21 ausübt. Dadurch ist zu jedem Zeitpunkt eine optimale Einwirkung des Instruments 21 angepasst an Bewegungs- oder Kraftparameter gewährleistet.

Es ist zusätzlich oder alternativ zu den beschriebenen Ausführungsformen möglich, eine Eindringtiefe und/oder Schnitttiefe einer Nadel, eines Messers bzw. einer nadelförmigen Instrumentenelektrode 24 oder einer Schneidelektrode in das zu behandelnde Gewebe G zu ermitteln und einen Betriebsparameter PB oder mehrere Betriebsparameter PB abhängig von der Eindringtiefe bzw. Schnitttiefe zu variieren. Beispielsweise kann eine dem elektrochirurgischen Instrument 21 bereitgestellte elektrische Leistung und/oder elektrische Spannung erhöht werden, wenn die Eindringtiefe bzw. Schnitttiefe zunimmt. Zusätzlich oder alternativ kann der Scheitelfaktor mit zunehmender Eindringtiefe und/oder Schnitttiefe verringert werden.

Die Eindringtiefe bzw. Schnitttiefe kann beispielsweise dadurch ermittelt werden, dass zunächst ein Kontakt zwischen einem distalen Ende des Instruments 21 (zum Beispiel Nadelelektrode oder Schneidelektrode) mit dem Gewebe G ermittelt wird. Dies kann beispielsweise dadurch erfolgen, dass sich an der Nadelelektrode oder der Schneidelektrode beim Kontakt mit dem Gewebe G ein Betriebsparameter PB verändert, beispielsweise weil dadurch ein Stromfluss durch das zu behandelnde Gewebe G erzeugt wird und/oder eine an der Instrumentenelektrode 24 (Nadelelektrode oder Schneidelektrode) anliegende Spannung sich verändert und insbesondere sinkt. Ausgehend von dieser Referenzposition kann eine mittels des Roboters 12 ausgeführte Bewegung erfasst werden und die Eindringtiefe bzw. Schnitttiefe in das zu behandelnde Gewebe G beschreiben.

Wie vorstehend erläutert, können die unterschiedlichen Handhabungsparameter PH ohne zusätzliche Sensorik am Instrument 21 durch die im Roboter 12 ohnehin bekannten Parameter, insbesondere Motorparameter, ermittelt werden. Optional können im Roboter 12 und insbesondere im Roboterarm 37 Sensoren, wie z.B. die erläuterten Motorsensoren 42, vorhanden sein. Derartige Robotersensoren sind allerdings nicht unmittelbar einem an der Halteeinrichtung 44 angeordneten Instrument 21 zugeordnet und sind im Roboter 12 für die Steuerung des Roboterarms 37 ohnehin vorhanden. Zusätzliche Sensoren, die dem Instrument 21 zugeordnet und dort oder an der Halteeinrichtung 44 angebracht werden, sind nicht erforderlich und bei den Ausführungsbeispielen auch nicht vorhanden.

Die Erfindung betrifft ein Chirurgiesystem 10 sowie ein Verfahren zu dessen Betrieb. Das Chirurgiesystem 10 hat eine Vorrichtung 11 und einen Roboter 12. Die Vorrichtung 11 weist ein Versorgungsgerät 20 und wenigstens ein an das Versorgungsgerät 20 angeschlossenes Instrument 21 auf, das zur Behandlung von biologischem Gewebe G eingerichtet ist. Das Instrument 21 oder zumindest eines der vorhandenen Instrumente 21 ist an eine Halteeinrichtung 44 des Roboters 12 angeordnet und kann mittels des Roboters 12 relativ zu dem zu behandelnden Gewebe G positioniert und/oder bewegt werden, wobei diese Relativbewegung und/oder Relativposition durch wenigstens einen Handhabungsparameter PH beschrieben ist. Zusätzlich oder alternativ kann ein Handhabungsparameter PH eine Kraft zwischen dem Instrument 21 und dem Gewebe G beschreiben. Zumindest einer der Handhabungsparameter PH oder mehrere Handhabungsparameter PH werden über eine Kommunikationseinrichtung der Vorrichtung 11 bereitgestellt, die abhängig von einem oder mehreren Handhabungsparametern PH einen oder mehrere Betriebsparameter PB für den Betrieb des Instruments 21 ermittelt und steuert oder regelt. Dadurch ist eine optimale Anpassung des Betriebs des Instruments 21 an die aktuell mittels des Roboters 12 auf das Instrument 21 ausgeübte Bewegung bzw. Kraft erreicht.

### Bezugszeichenliste:

- 10: Chirurgiesystem
- 11: Vorrichtung
- 12: Roboter
- 13: Kommunikationseinrichtung
- 14: Kommunikationsschnittstelle
- 15: Bedieneinrichtung

- 20: Versorgungsgerät
- 21: Instrument
- 22: Leitung
- 23: Stecker
- 24: Instrumentenelektrode
- 25: Neutralelektrode
- 26: Elektrodenleitung

- 31: Gerätesteuerung
- 32: Generator
- 33: Fluidquelle

- 37: Roboterarm
- 38: Armteil
- 39: Basis
- 40: Antriebseinheit
- 41: Elektromotor
- 42: Motorsensor
- 43: Robotersteuerung
- 44: Halteeinrichtung

- 50: Branche

- G: Gewebe
- I_{G}: Generatorstrom
- OP: Bedienperson
- P: Patient
- PB: Betriebsparameter
- PH: Handhabungsparameter
- U_{G}: Generatorspannung

## Patentansprüche

1. Chirurgiesystem (10) aufweisend:
- eine Vorrichtung (11) mit einem eine Gerätesteuerung (31) aufweisenden Versorgungsgerät (20) und mit wenigstens einem an das Versorgungsgerät (20) angeschlossenen Instrument (21), das zur Behandlung von biologischen Gewebe (G) dient, wobei die Gerätesteuerung (31) dazu eingerichtet ist, wenigstens einen Betriebsparameter (PB) für den Betrieb des wenigstens einen angeschlossenen Instruments (21) zu ermitteln und einzustellen,
- einen Roboter (12) mit einer Robotersteuerung (43) und mit einem bewegbaren Roboterarm (37), an dem eine Halteeinrichtung (44) zum Anordnen des Instruments (21) oder zumindest eines der Instrumente (21) vorhanden ist, wobei die Robotersteuerung (43) dazu eingerichtet ist, den Roboter (12) zu steuern, um das an der Halteeinrichtung (44) angeordnete Instrument (21) oder eines der an der Halteeinrichtung (44) angeordneten Instrumente (21) handzuhaben, wobei wenigstens ein im Roboter (12) vorgegebener und/oder ermittelter Handhabungsparameter (PH) eine Bewegung und/oder eine Position des betreffenden Instruments (21) relativ zu dem Gewebe (G) und/oder eine Kraft zwischen dem betreffenden Instrument (21) und dem Gewebe (G) beschreibt,
- eine Kommunikationseinrichtung (13), die dazu eingerichtet ist, den wenigstens eine Handhabungsparameter (PH) für die Gerätesteuerung (31) bereitzustellen, wobei die Gerätesteuerung (31) dazu eingerichtet ist, den wenigstens einen Betriebsparameter (PB) basierend auf dem Handhabungsparameter (PH) oder zumindest einem der Handhabungsparameter (PH) zu ermitteln.

2. Chirurgiesystem nach Anspruch 1, wobei die Kommunikationseinrichtung (13) wenigstens eine Kommunikationsschnittstelle (14) aufweist.

3. Chirurgiesystem nach Anspruch 2, wobei die Kommunikationsschnittstelle (14) oder eine der Kommunikationsschnittstellen (14) Bestandteil der Gerätesteuerung (31) und/oder der Robotersteuerung (43) ist.

4. Chirurgiesystem nach einem der vorhergehenden Ansprüche, wobei der Roboter (12) zur Positionierung und/oder Bewegung der Halteeinrichtung (44) wenigstens eine Antriebseinheit (40) aufweist, wobei jede Antriebseinheit (40) einen Elektromotor (41) aufweist.

5. Chirurgiesystem nach Anspruch 4, wobei wenigstens ein Motorparameter wenigstens eines Elektromotors (41) einen Handhabungsparameter (PH) definiert oder zur Ermittlung eines Handhabungsparameters (PH) in der Robotersteuerung (43) verwendet wird.

6. Chirurgiesystem nach Anspruch 5, wobei der wenigstens ein Motorparameter einer der nachfolgenden Parameter oder eine Kombination von mehreren der nachfolgenden Parameter ist:
- eine Motorspannung;
- ein Motorstrom;
- ein Motordrehmoment;
- eine Motordrehzahl;
- ein Motordrehwinkel;
- eine zeitliche Änderung irgendeines vorstehenden Motorparameters.

7. Chirurgiesystem nach einem der vorhergehenden Ansprüche, wobei der Roboter (12) wenigstens einen Sensor (42) aufweist, wobei der Sensor (42) dazu eingerichtet ist, einen Sensorwert bereitzustellen, der einen Handhabungsparameter (PH) definiert oder basierend auf dem ein Handhabungsparameter (PH) in der Robotersteuerung (43) ermittelbar ist.

8. Chirurgiesystem nach einem der vorhergehenden Ansprüche, wobei der Betriebsparameter (PB) oder zumindest einer der Betriebsparameter (PB) für das Instrument (21) oder für eines der vorhandenen Instrumente (21) gesteuert wird abhängig von einem die Bewegungsgeschwindigkeit des betreffenden Instruments (21) angebenden Handhabungsparameter (PH).

9. Chirurgiesystem nach einem der vorhergehenden Ansprüche, wobei der Betriebsparameter (PB) oder zumindest einer der Betriebsparameter (PB) für das Instrument (21) oder für eines der vorhandenen Instrumente (21) gesteuert wird abhängig von einem die Kraft zwischen dem betreffenden Instrument (21) und dem zu behandelnden Gewebe (G) angebenden Handhabungsparameter (PH).

10. Chirurgiesystem nach einem der vorhergehenden Ansprüche, wobei die Gerätesteuerung (31) und/oder die Robotersteuerung (43) dazu eingerichtet ist, einen Kontakt zwischen dem Instrument (21) oder einem der vorhandenen Instrumente (21) mit dem zu behandelnden Gewebe (G) zu ermitteln.

11. Chirurgiesystem nach Anspruch 10, wobei die Gerätesteuerung (31) und/oder die Robotersteuerung (43) dazu eingerichtet ist, eine seit dem Herstellen des Kontakts erfolgte Positionsänderung des Instruments (21) oder eines der vorhandenen Instrumente (21) zu ermitteln.

12. Chirurgiesystem nach Anspruch 11, wobei die Gerätesteuerung (31) und/oder die Robotersteuerung (43) dazu eingerichtet ist, basierend auf der Positionsänderung des Instruments (21) oder eines der vorhandenen Instrumente (21) eine Eindringtiefe in das zu behandelnde Gewebe (G) zu ermitteln.

13. Chirurgiesystem nach einem der vorhergehenden Ansprüche, wobei die Gerätesteuerung (31) und/oder die Robotersteuerung (43) dazu eingerichtet ist, eine Position eines beweglichen Teils (50) des Instruments (21) oder eines der vorhandenen Instrumente (21) zu ermitteln.

14. Verfahren zum Betreiben eines Chirurgiesystems (10), wobei das Chirurgiesystem (10) eine Vorrichtung (11) mit einem eine Gerätesteuerung (31) aufweisenden Versorgungsgerät (20) und mit wenigstens einem an das Versorgungsgerät (20) angeschlossenen Instrument (21) zur Behandlung von biologischen Gewebe (G) aufweist, wobei das Chirurgiesystem (10) außerdem einen Roboter (12) mit einer Robotersteuerung (43) und mit einem bewegbaren Roboterarm (37) aufweist, an dem eine Halteeinrichtung (44) zum Anordnen des Instruments (21) oder zumindest eines der Instrumente (21) vorhanden ist, und wobei das Chirurgiesystem (10) außerdem eine Kommunikationseinrichtung (13) aufweist, wobei das Verfahren umfasst:
- Ermitteln und Einstellen wenigstens eines Betriebsparameters (PB) für den Betrieb des wenigstens einen angeschlossenen Instruments (21) mittels der Gerätesteuerung (31),
- Steuern des Roboters (12) mittels der Robotersteuerung (43) zur Handhabung des an der Halteeinrichtung (44) angeordnete Instruments (21) oder eines der an der Halteeinrichtung (44) angeordneten Instrumente (21), wobei wenigstens ein im Roboter (12) vorgegebener oder ermittelter Handhabungsparameter (PH) eine Bewegung und/oder eine Position des betreffenden Instruments (21) relativ zu dem Gewebe (G) und/oder eine Kraft zwischen dem betreffenden Instrument (21) und dem Gewebe (G) beschreibt,
- Bereitstellen des wenigstens einen Handhabungsparameters (PH) für die Gerätesteuerung (31) mittels der Kommunikationseinrichtung (13),
- Ermitteln des wenigstens einen Betriebsparameters (PB) basierend auf dem Handhabungsparameter (PH) oder zumindest einem der Handhabungsparameter (PH) mittels der Gerätesteuerung (31).
